# EUROPEAN PATENT SPECIFICATION

(11) **EP 3 148 441 B1**
(45) Date of publication and mention of the grant of the patent: **11.07.2018**
(21) Application number: 15784754.2
(22) Date of filing: 26.05.2015
(51) Int. Cl.: A61B 6/12, A61B 6/00, A61M 25/00

(54) **CONTROL OF THE MOVEMENT AND IMAGE ACQUISITION OF AN X-RAY SYSTEM FOR A 3D/4D CO-REGISTERED RENDERING OF A TARGET ANATOMY**
STEUERUNG DER BEWEGUNGS- UND BILDERFASSUNG EINES RÖNTGENSYSTEMS FÜR EIN CO-REGISTRIERTES 3D/4D-RENDERING EINER ZIELANATOMIE
CONTRÔLE DU MOUVEMENT ET DE L'ACQUISITION D'IMAGE D'UN SYSTÈME À RAYONS X POUR UN RENDU 3D/4D CO-ENREGISTRÉ D'UNE ANATOMIE CIBLE

(30) Priority: 26.05.2014 US 201462003008 P
(43) Date of publication of application: 05.04.2017
(60) Divisional application: 18174832.8
(73) Proprietor: St. Jude Medical International Holding S.à r.l., 1417 Luxembourg (LU)
(72) Inventor: EICHLER, Uzi, 34657 Haifa (IL); HERSCOVICI, Adrian, 32762 Haifa (IL)
(74) Representative: Kramer Barske Schmidtchen Patentanwälte PartG mbB
(86) International application number: PCT/IB2015/001528
(87) International publication number: WO 2015/181636

(56) References cited:
- EP-A2- 1 901 232
- EP-A2- 1 944 733
- WO-A1-2006/116748
- US-A1- 2008 152 205

## Description

### CROSS-REFERENCE TO RELATED APPLICATIONS

This application claims priority to United States provisional application no. 62/003,008, filed 26 May 2014 (the '008 application). The '008 application is hereby incorporated by reference as though fully set forth herein.

### FIELD OF THE DISCLOSED TECHNIQUE

In general, the disclosed technique relates to medical imaging methods and systems. In particular, the disclosed technique relates to methods and systems for creating an anatomical model for navigating devices during medical procedures.

### BACKGROUND OF THE DISCLOSED TECHNIQUE

It is desirable to track the position of medical devices, such as catheters, as they are moved within the body so that, for example, drugs and other forms of treatment are administered at the proper location and so that medical procedures can be completed more efficiently and safely. Such navigational tracking can be accomplished by superimposing a still, pre-recorded representation of an anatomical target, such as a rotational angiogram of coronary vessels, on live images of a medical device, such as fluoroscopic images of a catheter or a biopsy needle. Rotational angiography is a medical imaging technique used to create a 3D model of an anatomical target using a plurality of two-dimensional images acquired with an image detector. Examples of rotational angiography systems include the DynaCT system made by Siemens AG and the Allura 3D Coronary Angiography by Philips Healthcare.

One drawback of using rotational angiography in combination with live imaging to provide anatomical visualization and medical device navigation is that such a procedure requires large doses fluoroscopy, including both x-ray radiation and contrast dye. For example, during a rotational angiography procedure, approximately 200 x-ray frames are taken during a 5-second C-arm rotation around an organ or region of interest. During this time, contrast dye must be injected into the organ to improve its visibility on x-ray. Such exposure to fluoroscopy is disadvantageous, however, because it subjects the patient and physician to undesirable levels of electromagnetic radiation.

Another drawback of using rotational angiography in combination with live imaging to provide anatomical visualization and medical device navigation is that there is often no good match between the pre-recorded anatomy shown in the rotational angiogram and the live anatomy shown on fluoroscopy, as the angiogram does not move in real time. Thus, many frames are required to compensate for the organ movement by gating for different organ phases, such as ECG gating for the cardiac cycle. However, the ECG signal is not always well correlated with the mechanical cardiac motion. The patient may also be required to hold his/her breath to eliminate movement artifacts due to respiration. Additionally, the anatomical region of interest must be positioned correctly in order to achieve adequate visualization.
WO 2006/116748 A1 relates to a system for navigating medical probes within anatomical organs by using a tracking subsystem configured for detecting the position of another medical device, wherein an imager positioning subsystem is configured for adjusting the field of view position based further on the other detected medical device position.
EP 1 901 232 A2 relates to a vascular treatment device for navigating through an occluded blood vessel.

### SUMMARY OF THE DISCLOSED TECHNIQUE

It is an object of the disclosed technique to provide a method and system for controlling the movement and image acquisition of an x-ray system used to generate an anatomic model. It is also an object of the disclosed technique to provide a method and system for optimizing the amount of fluoroscopy used to generate an anatomic model.

In accordance with the disclosed technique, there is thus provided a system for producing a model of an anatomical target area as defined in claim 1.

In accordance with another aspect of the disclosed technique, there is thus provided a method for producing a model of an anatomical target area as defined in claim 15.

The foregoing and other aspects, features, details, utilities, and advantages of the present disclosure will be apparent from reading the following description and dependent claims and from reviewing the accompanying drawings.

### BRIEF DESCRIPTION OF THE DRAWINGS

The disclosed technique will be understood and appreciated more fully from the following detailed description taken in conjunction with the drawings in which:
FIG. 1 is a schematic illustration of a system for producing a model of an anatomical target area using a real-time image of the body of a patient acquired by a moving imager, the position and orientation of the imager being determined according to the position and orientation of a medical device sensor, the system being constructed and operative in accordance with an embodiment of the disclosed technique;
FIG. 2 is a zoomed-in view of a schematic illustration of the system of FIG. 1 displaying an example of a position and orientation of the imager in relation to the medical device sensor, the system being constructed and operative in accordance with an embodiment of the disclosed technique;
FIG. 3 is a schematic illustration of a medical device comprising medical positioning system sensors and a dye injector device in accordance with an embodiment of the disclosed technique;
FIG. 4 is a schematic illustration of a method for producing a model of an anatomical target area using a real-time image of the body of a patient acquired by a moving imager, the position and orientation of the moving imager being determined according to the position and orientation of a medical device sensor, the system being constructed and operative in accordance with an embodiment of the disclosed technique;
FIGS. 5A and 5B are zoomed-in views of schematic illustrations of portions of the system of FIG. 1 displaying examples of the position and orientation of the imager in relation to a region of interest, the system being constructed and operative in accordance with an embodiment of the disclosed technique;
FIGS. 6 and 7 are zoomed-in views of schematic illustrations of a portion of the system of FIG. 1 displaying examples of a position and orientation of the imager used to provide minimal fluoroscopic exposure, the system being constructed and operative in accordance with an embodiment of the disclosed technique; and
FIG. 8A is a zoomed-in view of a three-dimensional schematic illustration of a vessel and associated sensors.
FIG. 8B-8F are schematic illustrations of simulated images of the vessel and sensors depicted in FIG. 8A taken from various different angles/orientations using the system of FIG. 1.

### DETAILED DESCRIPTION OF THE EMBODIMENTS

The disclosed technique overcomes the disadvantages of the prior art by controlling the movement and image acquisition of a X-ray system in order to optimize the amount of radiation and contrast dye used in generating models for anatomical visualization and navigation of medical devices.

The disclosed technique includes a method of optimizing x-ray and dye amounts used to create a 3D or 4D model of a target anatomy using the localization data provided by MediGuide™ Technology (e.g., patient reference sensors and/or magnetically tracked devices) to be used as co-registration information for navigating devices during medical procedures. The optimization is made possible by i) using a known location and orientation of a magnetically tracked device and/or patient reference sensor in the heart and other methods to define the region of interest location, ii) using a real time ECG and/or data from other patient reference sensors to characterize the mechanical cardiac motion and the motion caused by respiration, and iii) controlling the mechanical movement of an x-ray system (e.g., table, C-arm, source-to-image distance, image rotation), the image acquisition parameters (e.g., frame rate, zoom, or x-ray properties), and the trigger for image acquisition and dye release (e.g., based on biological signals such as respiration, patient movement, and cardiac cycle). Use of this method makes it possible to construct a 3D/4D model of an organ/region of interest using a limited amount of x-ray radiation (e.g., the amount of radiation required for a 2D cine loop currently used in numerous medical procedures). The resulting 3D/4D model can be used to navigate magnetically tracked devices without the need for more x-ray radiation or time consuming mapping procedures.

The term "cranio-caudal" axis herein below, refers to a longitudinal axis between the head of the patient and the toes of the patient. The term "medical device" herein below, refers to a vessel expansion unit such as a balloon catheter, stent carrying catheter, medical substance dispensing catheter, suturing catheter, guidewire, an ablation unit such as laser, cryogenic fluid unit, electric impulse unit, cutting balloon, rotational atherectomy unit (i.e., rotablator), directional atherectomy unit, transluminal extraction unit, drug delivery catheter, brachytherapy unit, intravascular ultrasound catheter, lead of a cardiac rhythm treatment (CRT) device, lead of an intra-body cardiac defibrillator (ICD) device, guiding device of a lead of a cardiac rhythm treatment device, guiding device of a lead of an intra-body cardiac defibrillator device, valve treatment catheter, valve implantation catheter, intra-body ultrasound catheter, intra-body computer tomography catheter, therapeutic needle, diagnostic needle, gastroenterology device (e.g., laparoscope, endoscope, colonoscope), orthopedic device, neurosurgical device, intra-vascular flow measurement device, intra-vascular pressure measurement device, intra-vascular optical coherence tomography device, intra-vascular near infrared spectroscopy device, intra-vascular infrared device (i.e., thermosensor), otorhinolaryngology precision surgery device, and the like.

The term "position" of an object herein below, refers to either the location or the orientation of the object, or both the location and orientation thereof. The term "magnetic region of interest" herein below, refers to a region of the body of the patient which has to be magnetically radiated by a magnetic field generator, in order for a medical positioning system (MPS) sensor to respond to the radiated magnetic field, and enable the MPS to determine the position of the tip of a medical device.

The term "image detector" herein below, refers to a device which produces an image of the visual region of interest. The image detector can be an image intensifier, flat detector (e.g., complementary metal-oxide semiconductor--CMOS), and the like. The term "magnetic coordinate system" herein below, refers to a three-dimensional coordinate system associated with the MPS. The term "3D optical coordinate system" herein below, refers to a three-dimensional coordinate system associated with a three-dimensional object which is viewed by the image detector. The term "2D optical coordinate system" herein below, refers to a two-dimensional coordinate system associated with the image detected by the image detector viewing the three-dimensional object.

The term "body region of interest" herein below, refers to a region of the body of a patient on which a therapeutic operation is to be performed. The term "visual region of interest" herein below, refers to a region of the body of the patient which is to be imaged by the moving imager. The term "image detector region of interest (ROI)" herein below, refers to different sizes of the detection region of the image detector. The image detector can detect the visual region of interest, either by utilizing the entire area of the image detector, or smaller areas thereof around the center of the image detector. The term "image detector ROI" refers to both an image intensifier and a flat detector.

The term "image rotation" herein below, refers to rotation of an image acquired by the image detector, performed by an image processor. The term "image flip" herein below, refers to a mirror image of the acquired image performed about an axis on a plane of the acquired image, wherein this axis represents the rotation of the acquired image about another axis perpendicular to the plane of the acquired image, relative to a reference angle (i.e., after performing the image rotation). For example, if the acquired image is rotated 25 degrees clockwise and an axis defines this amount of rotation, then the image flip defines another image obtained by rotating the acquired image by 180 degrees about this axis. In case no image rotation is performed, an image flip is performed about a predetermined axis (e.g., a substantially vertical axis located on the plane of the acquired image).

The term "moving image detector" herein below, refers to an image detector in which the image detector moves linearly along an axis substantially normal to the surface of the emitter, and relative to the emitter, in order to zoom-in and zoom-out.

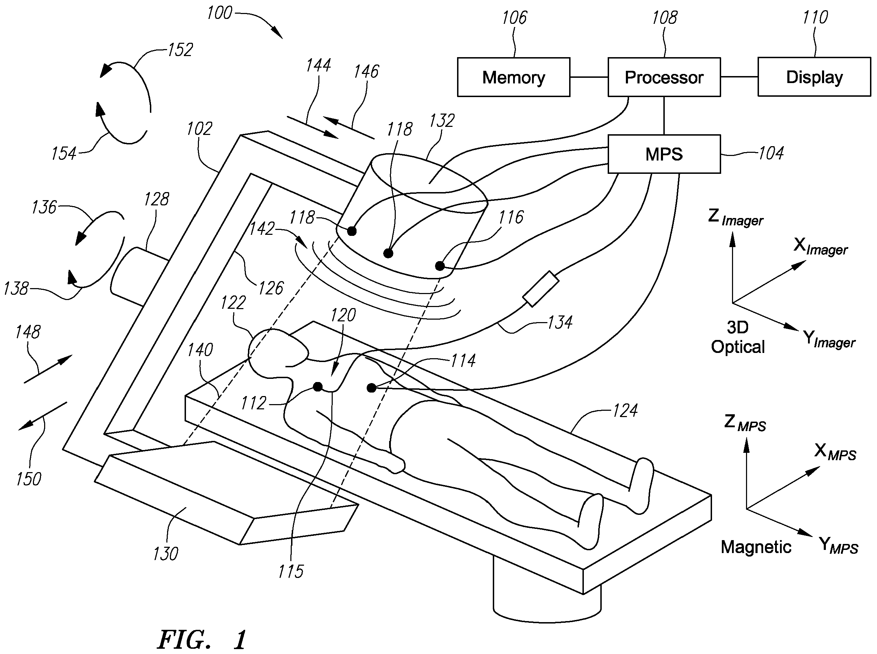

Reference is now made to FIG. 1, which is a schematic illustration of a system, generally referenced 100, for displaying a representation a distal portion of a medical device 134 on a real-time image of the body of a patient 122, acquired by a moving imager 102, the position being determined according to the position and orientation of an MPS sensor 112 or distal portion of the medical device 134, the system being constructed and operative in accordance with an embodiment of the disclosed technique. System 100, which is described in commonly assigned U.S. Patent Application Publication No. 2008/0183071, the entire disclosure of which is incorporated herein by reference, includes a moving imager 102, a medical positioning system (MPS) 104, a database 106, a processor 108, a display 110, MPS sensors 112, 114 and 116, a plurality of magnetic field generators 118 (i.e., transmitters).

Moving imager 102 is a device which acquires an image (not shown) of a body region of interest 120 of the body of a patient 122 lying on an operation table 124. Moving imager 102 includes a moving assembly 126, a moving mechanism 128, an emitter 130, and an image detector 132.

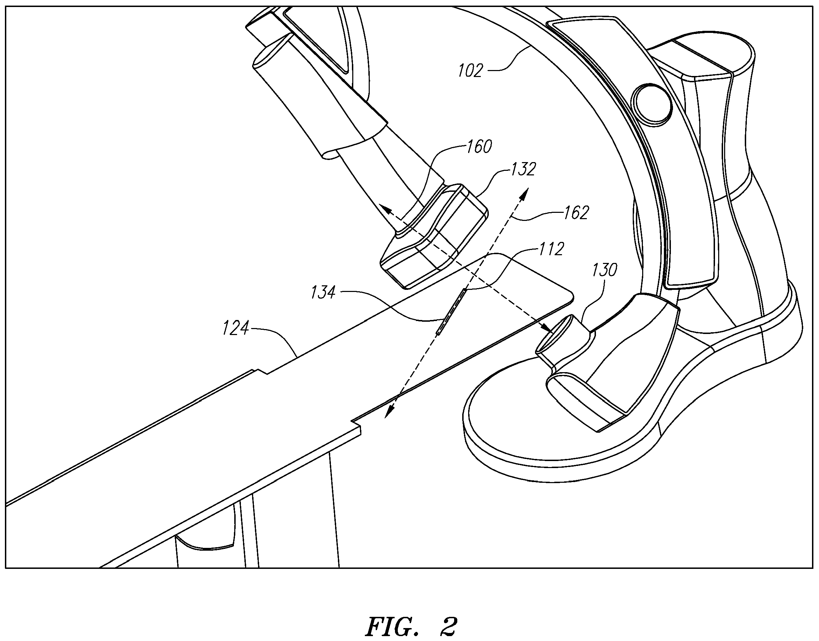

Moving imager 102 can operate based on X-rays, nuclear magnetic resonance, elementary particle emission, thermography, and the like. Moving imager 102 has at least one degree of freedom. In the example set forth in FIGS. 1 and 2, moving imager 102 is a C-arm imager. Emitter 130 and image detector 132 are coupled with moving assembly 126, such that emitter 130 is located at one side of patient 122 and image detector 132 is located at the opposite side of patient 122. Emitter 130 and image detector 132 are located on a radiation axis (not shown), wherein the radiation axis crosses the body region of interest 120.

The system can further include a user interface (e.g., a push button, joystick, foot pedal) coupled with the moving imager, to enable the physical staff to sequentially rotate the image acquired by the image detector, to flip the image at a given rotation angle, or set the ROI of the image detector. The moving imager is constructed such that the image indexes forward or backward by a predetermined amount, at every activation of the push button. This index can be for example, five degrees, thus enabling the moving imager to perform a maximum of seventy two image rotations (i.e., 360 divided by 5). Since the moving imager can produce one image flip for each image rotation, a maximum of hundred and forty four images (i.e., 72 times 2) can be obtained from a single image acquired by the image detector.

In an embodiment, magnetic field generators 118 are firmly coupled with image detector 132 (in other embodiments, magnetic field generators 118 can be located elsewhere, such as under the operation table 124). MPS sensor 112 is located at a distal portion of a medical device 134. MPS sensor 114 is attached to a substantially stationary location of the body of patient 122. Medical device 134 is inserted to the body region of interest 120. MPS sensors 112 and 114, and magnetic field generators 118 are coupled with MPS 104. Each of MPS sensors 112 and 114 can be coupled with MPS 104 either by a conductor or by a wireless link. Processor 108 is coupled with moving imager 102, MPS 104, database 106 and with display 110.

Moving imager 102 is associated with an X_{IMAGER}, Y_{IMAGER}, Z_{IMAGER} coordinate system (i.e., a 3D optical coordinate system). MPS 104 is associated with an X_{MPS}, Y_{MPS}, Z_{MPS} coordinate system (i.e., a magnetic coordinate system). The scaling of the 3D optical coordinate system is different than that of the magnetic coordinate system. Moving mechanism 128 is coupled with moving assembly 126, thereby enabling moving assembly 126 to rotate about the Y_{IMAGER} axis. Moving mechanism 128 rotates moving assembly 126 in directions designated by arrows 136 and 138, thereby changing the orientation of the radiation axis on the X_{IMAGER}-Z_{IMAGER} plane and about the Y_{IMAGER} axis. Moving mechanism 128 enables moving assembly 126 to rotate about the X_{IMAGER} axis. Moving mechanism 128 rotates moving assembly 126 in directions designated by arrows 152 and 154, thereby changing the orientation of the radiation axis on the Z_{IMAGER}-Y_{IMAGER} plane and about the X_{IMAGER} axis. Moving imager 102 can include another moving mechanism (not shown) coupled with moving imager 102, which can move moving imager 102 along the Y_{IMAGER} axis in directions designated by arrows 144 and 146 (i.e., along the cranio-caudal axis of patient 122). Moving imager 102 can include a further moving mechanism (not shown) coupled with moving imager 102, which can move moving imager 102 along the X_{IMAGER} axis in directions designated by arrows 148 and 150 (i.e., perpendicular to the cranio-caudal axis of patient 122).

Moving mechanism 128 or another moving mechanism (not shown) coupled with operation table 124, can enable relative movements between moving imager 102 and the body region of interest 120 along the three axes of the 3D optical coordinate system, in addition to rotations in directions 136, 138, 152 and 154. Each of emitter 130 and image detector 132 is constructed and operative by methods known in the art.

Image detector 132 can be provided with linear motion in directions toward and away from emitter 130, for varying the focal length of the image (i.e., in order to zoom-in and zoom-out). This zoom operation is herein below referred to as "physical zoom." In this case, system 100 further includes a detector moving mechanism (not shown) coupled with image detector 132, in order to impart this linear motion to image detector 132. The detector moving mechanism can be either motorized or manual. The term "physical zoom" herein below, applies to an image detector which introduces distortions in an image acquired thereby (e.g., an image intensifier), as well as an image detector which introduces substantially no distortions (e.g., a flat detector). MPS sensor 116 (i.e., image detector MPS sensor) can be firmly coupled with image detector 132 and coupled with MPS 104, in order to detect the position of image detector 132 along an axis (not shown) substantially normal to the surface of emitter 130, in the magnetic coordinate system.

Alternatively, image detector 132 can include a position detector (not shown) coupled with processor 108, to inform processor 108 of the current position of moving imager 102 relative to emitter 130. This position detector can be of a type known in the art, such as optic, sonic, electromagnetic, electric, mechanical, and the like. In case such a position detector is employed, processor 108 can determine the current position of moving imager 102 according to the output of the position detector, and MPS sensor 116 can be eliminated from system 100.

Alternatively, image detector 132 is substantially stationary relative to emitter 130 during the real-time operation of system 100. In this case, the physical zoom is performed by moving moving-assembly 126 relative to body region of interest 120, or by moving operation table 124. In this case, MPS sensor 116 can be eliminated from system 100. This arrangement is generally employed in mobile imagers, as known in the art. Alternatively, processor 108 can determine the physical zoom according to an input from the physical staff via the user interface. In this case too, MPS sensor 116 can be eliminated.

Additionally, moving imager 102 can perform a zoom operation which depends on an image detector ROI setting. In this case, an image processor (not shown) associated with moving imager 102, produces zoomed images of the acquired images, by employing different image detector ROI settings, while preserving the original number of pixels and the original dimensions of each of the acquired images.

It is noted that the physical zoom setting of image detector 132 is a substantially continuous function (i.e., the physical zoom can be set at any non-discrete value within a given range). The image detector ROI can be set either at one of a plurality of discrete values (i.e., discontinuous), or non-discrete values (i.e., continuous).

Magnetic field generators 118 are firmly coupled with image detector 132, in such a manner that magnetic field generators 118 do not physically interfere with radiations generated by image detector 132, and thus emitter 130 can direct a radiation at a field of view 140 toward the body region of interest 120, to be detected by image detector 132. In this manner, emitter 130 radiates a visual region of interest (not shown) of the body of patient 122. Image detector 132 produces an image output respective of the image of the body region of interest 120 in the 3D optical coordinate system. Image detector 132 sends the image output to processor 108 for display 110 to display the body region of interest 120. The location of MPS sensor 112 can be shown in the display.

Magnetic field generators 118 produce a magnetic field 142 toward the body region of interest 120, thereby magnetically radiating a magnetic region of interest (not shown) of the body of patient 122. Since magnetic field generators 118 are firmly coupled with image detector 132, the field of view 140 is included within magnetic field 142, no matter what the position of image detector 132. Alternatively, magnetic field 142 is included within field of view 140. In any case, body region of interest 120 is an intersection of field of view 140 and magnetic field 142. MPS 104 determines the position of the distal portion of medical device 134 (i.e., performs position measurements) according to the output of MPS sensor 112.

As a result of the direct and firm coupling of magnetic field generators 118 with image detector 132, the visual region of interest substantially coincides with the magnetic region of interest, and MPS sensor 112 responds to magnetic field 142 substantially at all times during the movements of moving imager 102. It is desirable to determine the position of the distal portion of medical device 134, while medical device 134 is inserted into any portion of the body of patient 122 and while moving imager 102 is imaging that same portion of the body of patient 122. Since magnetic field generators 118 are firmly coupled with moving imager 102 and move with it at all times, system 100 provides this capability. This is true for any portion of the body of patient 122 which moving imager 102 can move toward, in order to detect an image thereof.

Since magnetic field generators 118 are firmly coupled with moving imager 102, the 3D optical coordinate system and the magnetic coordinate system are firmly associated therewith and aligned together. Thus, when moving imager 102 moves relative to the body region of interest 120, magnetic field generators 118 move together with moving imager 102. The 3D optical coordinate system and the magnetic coordinate system are rigidly coupled. Therefore, it is not necessary for processor 108 to perform on-line computations for correlating the position measurements acquired by MPS 104 in the magnetic coordinate system, with the 3D optical coordinate system.

Thus, the position of MPS sensor 112 relative to the image of the body region of interest 120 detected by moving imager 102, can be determined without performing any real-time computations, such as transforming the coordinates according to a transformation model (i.e., transformation matrix), and the like. In this case, the transformation matrix for transforming a certain point in the magnetic coordinate system to a corresponding point in the 3D optical coordinate system, is a unity matrix.

It is noted that magnetic field generators 118 are located substantially close to that portion of the body of patient 122, which is currently being treated and imaged by moving imager 102. Thus, it is possible to use magnetic field generators which are substantially small in size and which consume substantially low electric power. This is true for any portion of the body of patient 122 which moving imager 102 can move toward, in order to detect an image thereof. This arrangement increases the sensitivity of MPS 104 to the movements of MPS sensor 112 within the body of patient 122, and reduces the cost, volume and weight of magnetic field generators 118.

Furthermore, this arrangement of magnetic field generators 118 provides the physical staff (not shown) a substantially clear view of body region of interest 120, and allows the physical staff a substantially easy reach to body region of interest 120. Since magnetic field generators 118 are firmly coupled with moving imager 102, any interference (e.g., magnetic, electric, electromagnetic) between MPS 104 and moving imager 102 can be identified beforehand, and compensated for during the operation of system 100.

It is further noted that the system can include MPS sensors, in addition to MPS sensor 112. It is noted that the magnetic field generators can be part of a transmitter assembly, which includes the magnetic field generators, a plurality of mountings for each magnetic field generator, and a housing to enclose the transmitter assembly components. The transmitter assembly can be for example, in an annular shape which encompasses image detector 132.

MPS 104 determines the viewing position value of image detector 132, according to an output of MPS sensor 114 (i.e., patient body MPS sensor), in the magnetic coordinate system, relative to the position of the body of patient 122. In this manner, processor 108 can compensate for the movements of patient 122 and of moving imager 102 during the medical operation on patient 122, according to an output of MPS 104, while processor 108 processes the images which image detector 132 acquires from body region of interest 120.

In case moving imager 102 is motorized, and can provide the position thereof to processor 108, directly, it is not necessary for processor 108 to receive data from MPS 104 respective of the position of MPS sensor 114, for determining the position of image detector 132. However, MPS sensor 114 is still necessary to enable MPS 104 to determine the position of the body of patient 122.

In an embodiment, processor 108 determines MPS data from MPS 104. The MPS data includes the position and/or orientation of the distal portion of medical device 134. The processor 108 determines such MPS data according to the output of MPS sensor 112, which can be positioned in a known anatomical location (e.g., coronary sinus) within body region of interest 120 using MPS 104. The processor 108 then uses the MPS data, in conjunction with known anatomical and/or physiological information, to control the position of moving imager 102.

Specifically, processor 108 can control movement of moving mechanism 128 or another moving mechanism (not shown), moving assembly 126, image detector 132, emitter 130, or operation table 124, so as to position the moving imager 102 at prescribed angles or orientations based on the position and orientation of the medical device or anatomical target area as detected by MPS sensor 112. Such prescribed angles or orientations can be determined by processor 108 based on a look-up table stored in memory 106. For example, a look-up table correlating a specified position of the distal portion of medical device 134 or MPS sensor 112 with a specified angle/orientation of moving imager 102 can be used by processor 108 to determine the optimal angle/orientation of moving imager 102. The look-up table can also include the associated anatomical location of MPS sensor 112, as well as the physiological state of the patient 122 (e.g., which phase of the cardiac and/or respiratory cycle the patient is in, as determined by patient reference sensors). The optimal angle/orientation of moving imager 102 can be that which best avoids the foreshortening effect of an organ in the x-ray image, thereby diminishing the number of x-ray frames required to adequately visualize the organ.

Referring to FIG. 2, a zoomed-in view of the position and orientation of moving imager 102 is shown with respect to the distal portion of medical device 134 and MPS sensor 112. Although it is contemplated that the distal portion of medical device 134 and MPS sensor 112 are located within a body of a patient, no patient is shown in FIG. 2 in order to more clearly illustrate the spatial relationship between moving imager 102 and the distal portion of medical device 134/MPS sensor 112. In the illustrated embodiment, moving imager 102 is positioned so that axis 160, between emitter 130 and image detector 132, is perpendicular to axis 162, the longitudinal axis of the distal portion of medical device 134 as defined by the orientation of MPS sensor 112. Moving imager 102 is also positioned so that emitter 130 and image detector 132 are centered on axis 162. It can be assumed that the coronary vessel in which the distal portion of medical device 134 resides is coaxial with the device and shares longitudinal axis 162. Thus, by positioning moving imager 102 so that axis 160 is perpendicular to axis 162, and by centering moving imager 102 on axis 162, a focused image of a substantial portion of a coronary vessel can be taken with minimal fluoroscopy and without the need for many additional frames.

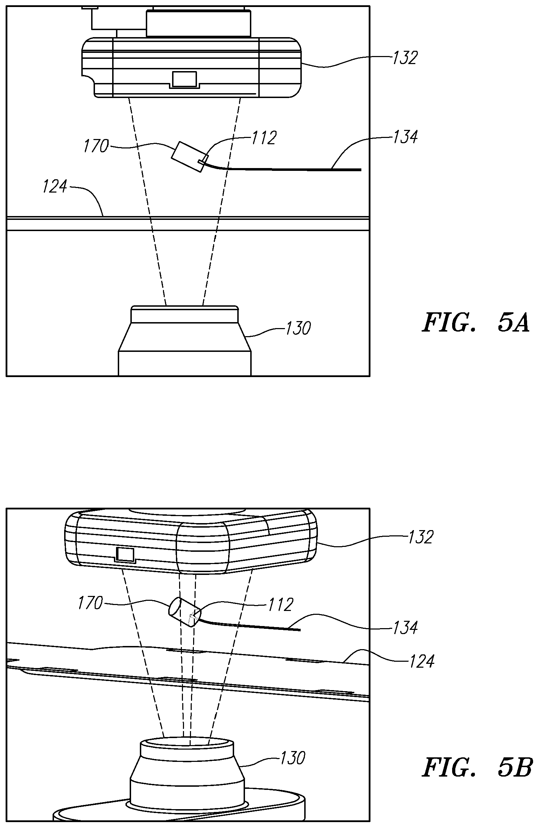

Referring to FIGS. 5A and 5B, the alignment of image detector 132 and emitter 130 is shown, along with ROI 170, represented here as a cylinder, in relation to MPS sensor 112. As shown in FIG. 5B, the base of the cylinder is concentric with MPS sensor 112 and aligned with its axis 162 (shown in FIG. 2). Optionally, the ROI 170 can be defined as a sphere, a cone, or another shape in place of a cylinder. It should be noted that the x-ray beam emitted from emitter 130 is wider than the volume of the volume of ROI 170.

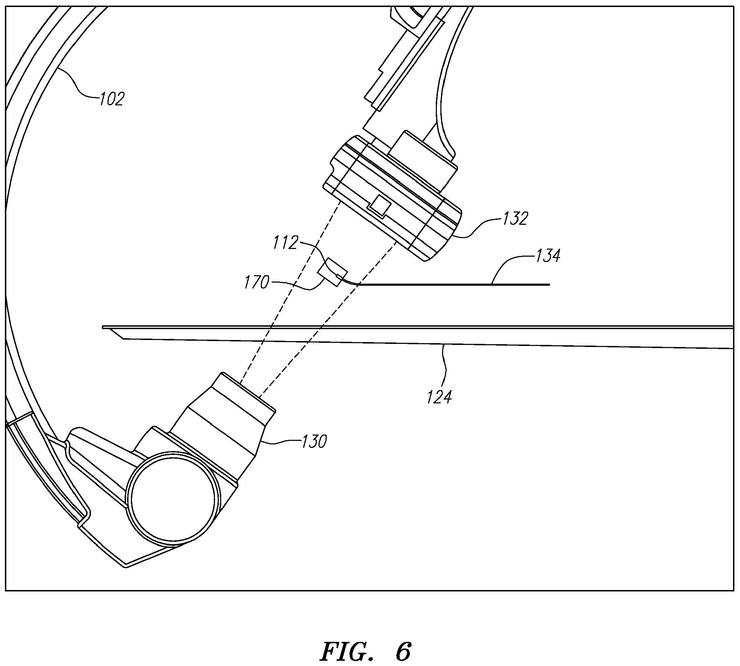

Referring to FIG. 6, the alignment of image detector 132 and emitter 130 is again shown with ROI 170. In this example, the imager 120 has been rotated 30 degrees caudally in order to minimize the area (e.g., on the body of the patient 122) that is exposed to the x-ray beam. The ROI 170 remains centered on and perpendicular to axis 160 (shown in FIG. 2) between the emitter 130 and the image detector 132.

In an embodiment, a 3D model of ROI 170 can be produced using images taken from at least two different angles/orientations of moving imager 102 (e.g., two angles separated by about 30-45 degrees). In another embodiment, a 4D model (e.g., real time) of ROI 170 can be produced using images taken from at least three different angles/orientations of moving imager 102.

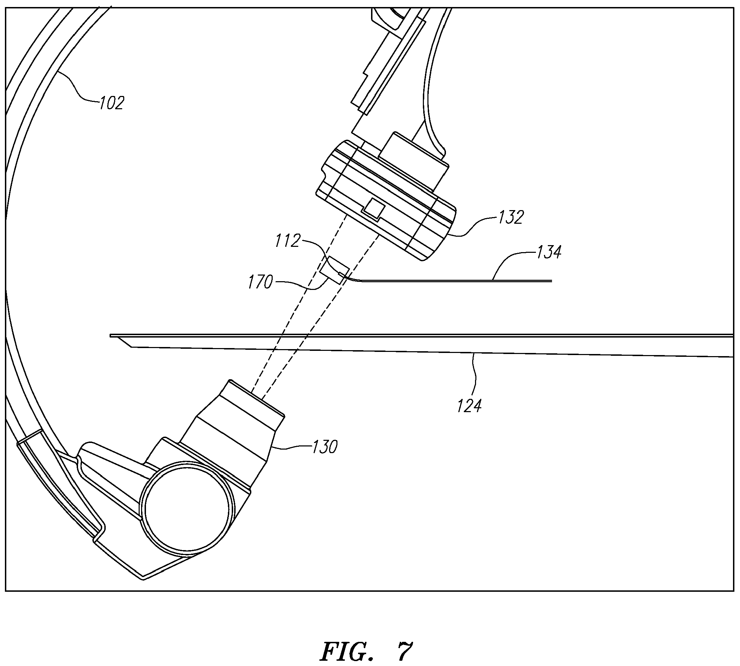

Referring to FIG. 7, the x-ray beam can be collimated to narrow in on ROI 170 and further limit fluoroscopic exposure. An emitter collimator (not shown) can be adjusted to fit the size of ROI 170, including movements due to the cardiac or respiration cycles, for example.

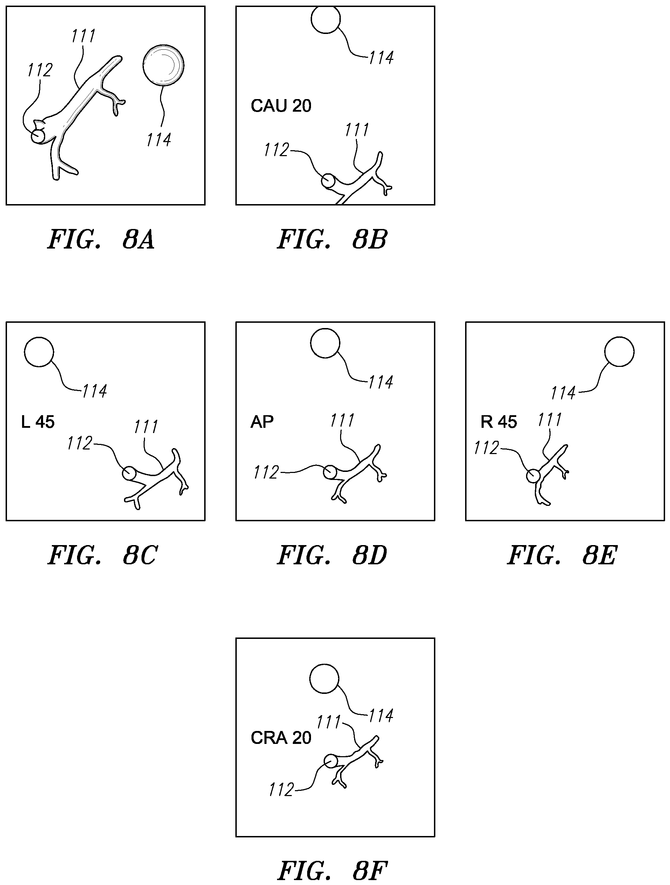

FIG. 8A is a zoomed-in view of a three-dimensional schematic representation of a branching blood vessel 111, such as a coronary vein, is shown in conjunction with the positions of the MPS sensors 112 and 114. FIGS. 8B-8F are schematic illustrations of simulated x-ray images of the vessel 111 and position sensors 112 and 114 taken from various different angles/orientations of the imager 102. Each simulated x-ray image shows the vessel 111 and the positions of the MPS sensors 112 and 114 as viewed from a specified position of the imager 102. For example, FIG. 8D is a simulated anterior-posterior x-ray image, defining a starting position of the imager 102. FIG. 8B shows a simulated x-ray image taken when the imager 102 has been rotated 20 degrees caudally relative to the starting position. FIG. 8C shows a simulated x-ray image taken when the imager 102 has been rotated 45 degrees to the left relative to the starting position. FIG. 8E shows a simulated x-ray image taken when the imager 102 has been rotated 45 degrees to the right relative to the starting position. FIG. 8F shows a simulated x-ray image taken when the imager 102 has been rotated 20 degrees cranially relative to its starting position.

Referring again to FIG. 1, processor 108 can use MPS data to control the activation or deactivation timing of emitter 130 and/or a contrast dye injector device 115. Contrast dye injector device 115 can be coupled directly or indirectly to processor 108 and/or moving imager 102. Contrast dye injector device 115 can be located at the distal portion of medical device 134, as shown in FIG. 1.

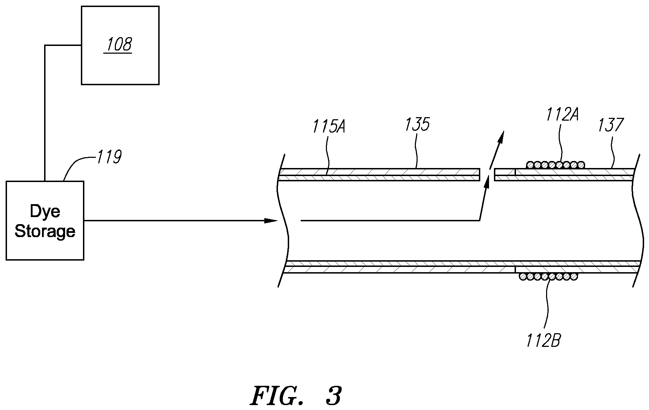

In the embodiment shown in FIG. 3, a contrast dye injector device 115A can be located within a catheter 135 with MPS sensors 112A and 112B attached to the distal end 137. In this embodiment, MPS sensors 112A and 112B are magnetic coils. Contrast dye injector device 115 or 115A can be coupled to a dye storage unit 119, which in turn can be coupled to processor 108.

Similar to the aforementioned look-up tables correlating a position of MPS sensor 112 with an angle of moving imager 102, look-up tables correlating a specified position of MPS sensor 112 (or 112A or 112B) with an activation/deactivation timing signal for emitter 130 can be stored in memory 106 and used by processor 108 to create optimally-timed x-ray acquisition, thereby limiting fluoroscopic exposure. Likewise, look-up tables correlating a specified position of MPS sensor 112 (or 112A or 112B) with an activation/deactivation timing signal for the release of dye from contrast dye injector device 115 or 115A can be stored in memory 106 and used by processor 108 to create optimally-timed x-ray acquisition, thereby limiting fluoroscopic exposure.

In an embodiment, input from a patient reference sensor, such as MPS sensor 114, for example, can be used by processor 108 to control positioning, orientation, and/or activation of moving imager 102. Examples of input from patient reference sensors include data regarding the patient's cardiac or respiratory cycle.

In an embodiment, moving imager 102 can be positioned so that the source-to-image distance (SID) between the emitter 130 and the MPS sensor 112 is predefined based on the MPS data.

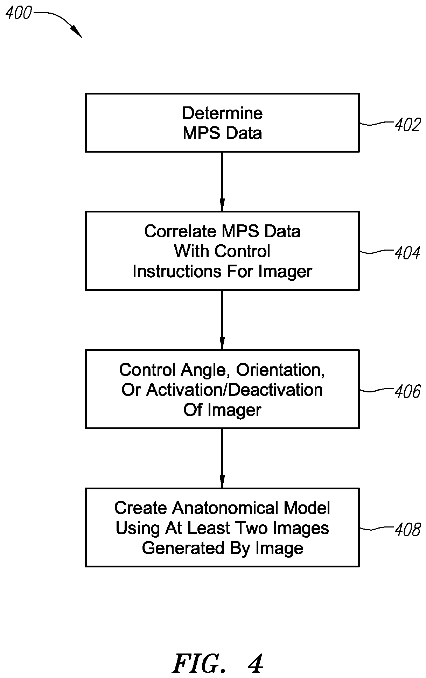

Reference is now made to FIG. 4, which is a schematic illustration of a method 400 for producing an anatomical model by controlling the position, orientation, and image acquisition of an x-ray imaging system, the system being constructed and operative in accordance with an embodiment of the disclosed technique. At step 402, MPS data is determined. At least one MPS sensor image of at least one MPS sensor (e.g., MPS sensor 112 located at the distal portion of medical device 134) is acquired by a moving imager, as described above with respect to FIG. 1. The output of the MPS sensor determines the MPS data, which is the position and/or orientation of a medical device coupled to the MPS sensor.

Next, at step 404, the MPS data is correlated with control instructions for the moving imager. This step can be performed by a processor using look-up tables stored in a memory. Such look-up tables can correlate a specified position of a medical device or a MPS sensor with i) a specified angle of the moving imager, ii) a specified activation or deactivation timing signal for the moving imager, or iii) a specified activation or deactivation timing signal for a dye injector device (e.g., contrast dye injector device 115 or 115A shown in FIGS. 1 and 3, respectively). The look-up tables can also include the associated anatomical location of the MPS sensor, as well as the physiological state of the patient (e.g., which phase of the cardiac and/or respiratory cycle the patient is in, as determined by patient reference sensors). These correlations are used to determine control instructions that the processor provides to the moving imager.

Next, at step 406, the angle, orientation or activation/deactivation status of the moving imager are controlled by the processor based on the information derived from the look-up tables. For example, based on the specified position of an MPS sensor, the moving imager may be positioned at a prescribed angle or orientation. Moreover, the moving imager, or an emitter portion of the moving imager, may be activated or deactivated based on the MPS data. Finally, a dye injector device may be activated or deactivated based on the MPS data. In addition to MPS data, data from one or more patient reference sensors can be used to control the angle, orientation or activation/deactivation status of the moving imager.

Finally, at step 408, an anatomical model of the target area can be created using at least two images generated by the moving imager. For example, at least two 2D images generated by the moving imager can be used to create a 3D anatomical model, and at least three 2D images generated by the moving imager can be used to create a 4D (e.g., real time) anatomical model.

It should be noted that the method 400 may include a co-registering step (not shown) in which the MPS coordinates of the MPS sensor are co-registered with imaging coordinates of the MPS sensor. This step may be omitted when magnetic field generators 118 are firmly coupled with moving imager 102, and the 3D optical coordinate system and the magnetic coordinate system are firmly associated therewith and aligned together, as described above with respect to FIG. 1.

The anatomical model produced according to method 400 can be displayed on display 110, shown in FIG. 1. The location of the MPS sensor (e.g., MPS sensor 112 shown in FIG. 1) can be shown in the displayed anatomical model.

Method 400 may further include an optional step (not shown) in which a user can control moving imager 102 via a user interface.

By using MPS data to determine and produce the optimal angle/orientation of the moving imager 102 and the optimal timing of image acquisition, clear visualization of the anatomical target area can be attained with minimal fluoroscopic exposure. The present inventors have estimated that use of the above described method can reduce fluoroscopy exposure by about 90% compared to prior art 3D visualization techniques.

Although embodiments of an articulation support member for a deflectable introducer have been described above with a certain degree of particularity, those skilled in the art could make numerous alterations to the disclosed embodiments without departing from the spirit or scope of this disclosure. All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the devices. Joinder references (e.g., affixed, attached, coupled, connected, and the like) are to be construed broadly and can include intermediate members between a connection of elements and relative movement between elements. As such, joinder references do not necessarily infer that two elements are directly connected and in fixed relationship to each other. It is intended that all matter contained in the above description or shown in the accompanying drawings shall be interpreted as illustrative only and not limiting. Changes in detail or structure can be made without departing from the spirit of the disclosure.

Any patent, publication, or other disclosure material, in whole or in part, that is said to be incorporated by reference herein is incorporated herein only to the extent that the incorporated materials does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Various embodiments have been described above to various apparatuses, systems, and/or methods. Numerous specific details have been set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated above are non-limiting examples, and thus it can be appreciated that the specific structural and functional details disclosed above may be representative and do not necessarily limit the scope of the embodiments.

Reference throughout the specification to "various embodiments," "some embodiments," "one embodiment," or "an embodiment", or the like, means that a particular feature, structure, or characteristic described in connection with the embodiment is included in at least one embodiment. Thus, appearances of the phrases "in various embodiments," "in some embodiments," "in one embodiment," or "in an embodiment", or the like, in places throughout the specification are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features, structures, or characteristics of one or more other embodiments without limitation given that such combination is not illogical or non-functional.

It will be appreciated that the terms "proximal" and "distal" have been used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" have been used above with respect to the illustrated embodiments. However, surgical instruments may be used in many orientations and positions, and these terms are not intended to be limiting and absolute.

## Claims

1. A system for producing a model of an anatomical target area, the system comprising:
a medical positioning system (104) comprising a medical device sensor (112); and
a processor (108) comprising circuitry configured to electrically communicate with the medical positioning system (104) and with an imager (102) configured to generate an image of the anatomical target area, the processor (108) configured to: i) determine position data from the medical positioning system (104), the position data comprising a position and/or an orientation of a medical device (134) according to an output of the medical device sensor (112), ii) control the imager (102) based on the position data, and iii) create the model of the anatomical target area using at least two images generated by the imager (102); **characterised in that** the medical device (134) comprises a contrast dye injector device (115) configured to deliver a contrast dye to the anatomical target area; and that the processor (108) is further configured to control delivery of the contrast dye from the contrast dye injector device (115) based on the position data.

2. The system of claim 1, further comprising a memory (106) comprising circuitry to electrically communicate with the processor (108), the medical positioning system (104), and the imager (102), the memory (106) configured to store a look-up table correlating the position data with control instructions for the imager (102).

3. The system of claim 1 or 2, wherein the medical positioning system is configured to determine medical positioning system coordinates of the medical device sensor (112) within a first coordinate system; wherein the imager (102) is configured to determine imaging coordinates of the medical device sensor (112) within a second coordinate system; and wherein the processor (108) is configured to co-register the first and second coordinate systems.

4. The system of any one of claims 1 to 3, wherein the model is a three-dimensional model.

5. The system of any one of claims 1 to 4, wherein the processor (108) is further configured to control an angle or an orientation of the imager (102) or a portion of the imager (102).

6. The system of claim 5, wherein the imager (102) comprises a fluoroscope, and wherein the portion of the imager (102) comprises a C-arm.

7. The system of claim 5, wherein the imager (102) comprises an emitter (130), a detector (132), and an axis between the emitter (130) and the detector (132); and wherein the axis between the emitter (130) and the detector (132) is perpendicular to a longitudinal axis of at least one of the medical device (134) or the medical device sensor (112).

8. The system of claim 1, wherein the processor (108) is further configured to control at least one of the following: an operation table position or tilt, a source-to-image distance between an emitter (130) and an image detector (132), an image rotation, a frame rate, or a physical zoom of the imager (102).

9. The system of claim 1, wherein the processor (108) is further configured to control the imager by controlling activation or deactivation timing of the imager (102).

10. The system of claim 1, wherein the processor (108) is further configured to i) determine movement data from a patient reference sensor, the movement data comprising information about real time phasic movement of the anatomical target area, and ii) control the imager (102) based on the movement data.

11. The system of claim 10, wherein the real time phasic movement of the anatomical target area comprises cardiac movement due to at least one of a cardiac cycle or a respiration cycle.

12. The system of claim 10, wherein the processor (108) is further configured to control delivery of the contrast dye from the contrast dye injector device (115) based on the movement data.

13. The system of any one of claims 1 to 12, further configured to produce a four-dimensional model of the anatomical target area.

14. The system of any one of claims 1 to 13, further comprising a user interface in communication with the processor (108), the user interface configured to receive control instructions for the imager (102) from a user.

## Patentansprüche

1. System zum Erzeugen eines Modells eines anatomischen Zielbereichs, wobei das System aufweist:
ein medizinisches Positionsbestimmungssystem (104) mit einem medizinischen Vorrichtungssensor (112); und
einen Prozessor (108) mit einer Schaltung, die konfiguriert ist zur elektrischen Kommunikation mit dem medizinischen Positionsbestimmungssystem (104) und mit einem Bildgeber (102), der konfiguriert ist zum Erzeugen eines Bilds des anatomischen Zielbereichs, wobei der Prozessor (108) konfiguriert ist zum: i) Bestimmen von Positionsdaten von dem medizinischen Positionsbestimmungssystem (104), wobei die Positionsdaten eine Position und/oder eine Orientierung der medizinischen Vorrichtung (134) aufweisen, gemäß einer Ausgabe des medizinischen Vorrichtungssensors (112), ii) Steuern des Bildgebers (102) basierend auf den Positionsdaten, und iii) Erzeugen des Modells des anatomischen Zielbereichs unter Verwendung von mindestens zwei Bildern, die von dem Bildgeber (102) erzeugt sind; **dadurch gekennzeichnet, dass**
die medizinische Vorrichtung (134) eine Injektionsvorrichtung (115) für Kontrastmittel aufweist, die konfiguriert ist zum Liefern eines Kontrastmittels an den anatomischen Zielbereich; und
der Prozessor (108) ferner konfiguriert ist zur Steuerung der Lieferung des Kontrastmittels von der Injektionsvorrichtung (115) für Kontrastmittel basierend auf den Positionsdaten.

2. System nach Anspruch 1, ferner mit einem Speicher (106), der eine Schaltung aufweist zur elektrischen Kommunikation mit dem Prozessor (108), dem medizinischen Positionsbestimmungssystem (104) und dem Bildgeber (102), wobei der Speicher (106) konfiguriert ist zum Speichern einer Nachschlagetabelle, die die Positionsdaten mit den Steuerungsanweisungen für den Bildgeber (102) korreliert.

3. System nach Anspruch 1 oder 2, bei dem das medizinische Positionsbestimmungssystem konfiguriert ist zum Bestimmen medizinischer Positionsbestimmungssystemkoordinaten des medizinischen Vorrichtungssensors (112) innerhalb eines ersten Koordinatensystems; wobei der Bildgeber (102) konfiguriert ist zum Bestimmen von Bildgebungskoordinaten des medizinischen Vorrichtungssensors (112) innerhalb eines zweiten Koordinatensystems; und wobei der Prozessor (108) konfiguriert ist, um das erste und zweite Koordinatensystem miteinander zu registrieren.

4. System nach einem der Ansprüche 1 bis 3, bei dem das Modell ein dreidimensionales Modell ist.

5. System nach einem der Ansprüche 1 bis 4, bei dem der Prozessor (108) ferner konfiguriert ist zum Steuern eines Winkels oder einer Orientierung des Bildgebers (102) oder eines Teils des Bildgebers (102).

6. System nach Anspruch 5, bei dem der Bildgeber (102) ein Fluoroskop aufweist und der Teil des Bildgebers (102) einen C-Arm aufweist.

7. System nach Anspruch 5, bei dem der Bildgeber (102) einen Emitter (130), einen Detektor (132) und eine Achse zwischen dem Emitter (130) und dem Detektor (132) aufweist; und bei dem die Achse zwischen dem Emitter (130) und dem Detektor (132) senkrecht zu einer Längsachse von mindestens einem von der medizinischen Vorrichtung (134) oder dem medizinischen Vorrichtungssensor (112) ist.

8. System nach Anspruch 1, bei dem der Prozessor (108) ferner konfiguriert ist zum Steuern von mindestens einem von dem Folgenden: einer Operationstischposition oder Neigung, einem Quelle-zu-Bild-Abstand zwischen einem Emitter (130) und einem Bilddetektor (132), einer Bilddrehung, einer Rahmenrate oder eines physikalischen Zoom des Bildgebers (102).

9. System nach Anspruch 1, bei dem der Prozessor (108) ferner konfiguriert ist zum Steuern des Bildgebers durch Steuerung von einer Aktivierungs- oder Deaktivierungszeit des Bildgebers (102).

10. System nach Anspruch 1, bei dem der Prozessor (108) ferner konfiguriert ist zum i) Bestimmen von Bewegungsdaten von einem Patientenreferenzsensor, wobei die Bewegungsdaten Information über eine Echtzeitphasenbewegung des anatomischen Zielbereichs aufweisen, und ii) Steuern des Bildgebers (102) basierend auf den Bewegungsdaten.

11. System nach Anspruch 10, bei dem die Echtzeitphasenbewegung des anatomischen Zielbereichs eine Herzbewegung aufweist aufgrund von mindestens einem Herzzyklus oder einem Atemzyklus.

12. System nach Anspruch 10, bei dem der Prozessor (108) ferner konfiguriert ist zum Steuern der Lieferung des Kontrastmittels von der Injektionsvorrichtung (115) für Kontrastmittel basierend auf den Bewegungsdaten.

13. System nach einem der Ansprüche 1 bis 12, ferner konfiguriert zum Erzeugen eines vierdimensionalen Modells des anatomischen Zielbereichs.

14. System nach einem der Ansprüche 1 bis 13, ferner mit einer Benutzerschnittstelle in Kommunikation mit dem Prozessor (108), wobei die Benutzerschnittstelle konfiguriert ist zum Empfangen von Steuerungsanweisungen für den Bildgeber (102) von einem Benutzer.

## Revendications

1. Système de production d'un modèle d'une zone cible anatomique, le système comprenant :
un système de positionnement médical (104) comprenant un capteur de dispositif médical (112) ; et
un processeur (108) comprenant une circuiterie configurée pour communiquer électriquement avec le système de positionnement médical (104) et avec un imageur (102) configuré pour générer une image de la zone cible anatomique, le processeur (108) étant configuré pour i) déterminer les données de position issues du système de positionnement médical (104), les données de position comprenant une position et/ou une orientation d'un dispositif médical (134) selon une sortie du capteur de dispositif médical (112), ii) commander l'imageur (102) sur la base des données de position et iii) créer le modèle de la zone cible anatomique en utilisant au moins deux images générées par l'imageur (102) ;
**caractérisé en ce que** :
le dispositif médical (134) comprend un dispositif injecteur de colorant de contraste (115) configuré pour délivrer un colorant de contraste à la zone cible anatomique ; et
le processeur (108) est en outre configuré pour commander la fourniture du colorant de contraste issu du dispositif injecteur de colorant de contraste (115) sur la base des données de position.

2. Système selon la revendication 1, comprenant en outre une mémoire (106) comprenant une circuiterie pour communiquer électriquement avec le processeur (108), le système de positionnement médical et l'imageur (102), la mémoire (106) étant configurée pour stocker une table de consultation corrélant les données de position avec les instructions de commande pour l'imageur (102).

3. Système selon la revendication 1 ou 2, dans lequel le système de positionnement médical est configuré pour déterminer des coordonnées dans le système de positionnement médical du capteur de dispositif médical (112) dans un premier système de coordonnées ; dans lequel l'imageur (102) est configuré pour déterminer des coordonnées d'imagerie du capteur de dispositif médical (112) dans un second système de coordonnées ; et dans lequel le processeur (108) est configuré pour co-enregistrer le premier et le second système de coordonnées.

4. Système selon l'une quelconque des revendications 1 à 3, dans lequel le modèle est un modèle tridimensionnel.

5. Système selon l'une quelconque des revendications 1 à 4, dans lequel le processeur (108) est en outre configuré pour commander un angle ou une orientation de l'imageur (102) ou d'une partie de l'imageur (102).

6. Système selon la revendication 5, dans lequel l'imageur (102) comprend un fluoroscope et dans lequel la partie de l'imageur (102) comprend un bras C.

7. Système selon la revendication 5, dans lequel l'imageur (102) comprend un émetteur (130), un détecteur (132) et un axe entre l'émetteur (130) et le détecteur (132) ; et dans lequel l'axe entre l'émetteur (130) et le détecteur (132) est perpendiculaire à un axe longitudinal d'au moins l'un du dispositif médical (134) ou du capteur de dispositif médical (112).

8. Système selon la revendication 1, dans lequel le processeur (108) est en outre configuré pour commander au moins l'une des situations suivantes : une position ou un basculement de la table d'opération, une distance source-image entre un émetteur (130) et le détecteur d'image (132), une rotation d'image, un débit de trames ou un zoom physique de l'imageur (102).

9. Système selon la revendication 1, dans lequel le processeur (108) est en outre configuré pour commander l'imageur en commandant la synchronisation d'activation ou de désactivation de l'imageur (102).

10. Système selon la revendication 1, dans lequel le processeur (108) est en outre configuré pour i) déterminer des données de mouvement issues d'un capteur de référence de patient, les données de mouvement comprenant des informations sur le mouvement phasique en temps réel de la zone cible anatomique et ii) commander l'imageur (102) sur la base des données de mouvement.

11. Système selon la revendication 10, dans lequel le mouvement phasique en temps réel de la zone cible anatomique comprend un mouvement cardiaque dû à au moins l'un d'un cycle cardiaque ou d'un cycle de respiration.

12. Système selon la revendication 10, dans lequel le processeur (108) est en outre configuré pour commander la fourniture du colorant de contraste venant du dispositif injecteur de colorant de contraste (115) sur la base des données de mouvement.

13. Système selon l'une quelconque des revendications 1 à 12, configuré en outre pour produire un modèle quadridimensionnel de la zone cible anatomique.

14. Système selon l'une quelconque des revendications 1 à 13, comprenant en outre une interface utilisateur en communication avec le processeur (108), l'interface utilisateur étant configurée pour recevoir des instructions de commande d'un utilisateur pour l'imageur (102).
